# EUROPÄISCHE PATENTSCHRIFT

(11) **EP 1 863 421 B1**
(45) Veröffentlichungstag und Bekanntmachung des Hinweises auf die Patenterteilung: **04.05.2022**
(21) Anmeldenummer: 06723303.1
(22) Anmeldetag: 09.03.2006
(51) Int. Cl.: A61F 9/008, G02B 7/10, G02B 26/10

(54) **Laserchirurgisches System**
System for Laser Surgery
Système de chirurgie par laser

(30) Priorität: 26.03.2005 DE 102005013949
(43) Veröffentlichungstag der Anmeldung: 12.12.2007
(73) Patentinhaber: Carl Zeiss Meditec AG, 07745 Jena (DE)
(72) Erfinder: HANFT, Marco, 07743 Jena (DE); MÜHLHOFF, Dirk, 07751 Kunitz (DE); BISCHOFF, Mark, 99334 Elleben OT Riechheim (DE); GERLACH, Mario, 07768 Altenberga (DE)
(74) Vertreter: Beck, Bernard
(86) Internationale Anmeldenummer: PCT/EP2006/002150
(87) Internationale Veröffentlichungsnummer: WO 2006/102971

(56) Entgegenhaltungen:
- EP-A- 0 960 609
- EP-A2- 1 447 064
- WO-A-03/068051
- WO-A1-2005/011547
- GB-A- 1 512 037
- US-A- 4 554 666
- US-A- 5 997 141
- US-A1- 2004 243 112

## Beschreibung

Die vorliegende Erfindung bezieht sich auf ein Laserchirurgisches System beinhaltend eine Scanvorrichtung zur Fokussierung eines Strahlenbündels in vorgegebene Bereiche eines vorgegebenen Volumens sowie ein Verfahren zur Fokussierung eines Strahlenbündels in vorgegebene Bereiche eines Volumens.

Die Fokussierung eines Strahlenbündels in unterschiedliche vorgegebene Bereiche eines Volumens, das heißt sowohl in einer Richtung lateral bzw. quer zu dem Strahlenbündel als auch in einer Richtung entlang des Strahlenbündels, spielt in einer Reihe von optischen Vorrichtungen bzw. bei einer Reihe von optischen Verfahren eine wichtige Rolle. Unter Strahlenbündeln werden im folgenden insbesondere auch von Lasern abgegebene Laserstrahlen verstanden.

Beispielsweise werden Laserstrahlen in der Ophthalmologie eingesetzt, um eine Fehlsichtigkeit des menschlichen Auges durch einen laserchirurgischen Eingriff an der Cornea des Auges zu korrigieren. Von besonderer Bedeutung ist dabei das unter der Bezeichnung "LASIK" (Laser in situ Keratomileusis) bekannte Verfahren, bei dem mittels eines gepulsten Laserstrahls Material nicht an der Oberfläche der Cornea, sondern im Inneren der Cornea ablatiert, d.h. abgetragen, wird. Dazu wird an der äußeren Oberfläche der Cornea ein auch als "Flap" bezeichneter, klappenartiger Deckel gebildet, dessen Dicke wesentlich geringer ist als die Dicke der Cornea. Zur eigentlichen Ablationsbehandlung wird dieser Deckel weggeklappt, woraufhin von der Oberfläche des nun freigelegten Bereichs mittels eines gepulsten Laserstrahls Material zur Korrektur der Fehlsichtigkeit abgetragen wird.

Danach wird der Deckel auf die ablatierte Oberfläche zurückgeklappt.

Um den Deckel in genau definierter Tiefe in der Cornea möglichst schonend und präzise herstellen zu können, ist vorgeschlagen worden, Femtosekundenlaserpulse, d.h. Laserpulse mit Pulsbreiten kleiner als 10⁻¹²s, zu verwenden. Mittels solcher Pulse können in der Cornea optische Durchbrüche, auch Photodisruptionen genannt, erzeugt werden, die lokal begrenzt sind und eine Ausdehnung von nur wenigen Mikrometern aufweisen. Durch eine dichte Positionierung einer Vielzahl solcher optischer Durchbrüche an genau vorgegebenen Stellen kann der Deckel sehr exakt gebildet werden kann. Eine wesentliche Voraussetzung für die genaue Bildung des Deckels ist dabei die exakte Positionierung des Fokus des verwendeten gepulsten Laserstrahls nicht nur in lateraler Richtung, sondern vor allem auch in der Tiefe der Cornea und damit in Ausbreitungsrichtung des Laserstrahls.

In US 2003/0053219 A1 ist ein Zoomlinsensystem beschrieben, das zur Benutzung in der Chirurgie vorgesehen ist. Zur Fokussierung in verschiedene Tiefen, das heißt in Richtung des Laserstrahls, wird eine Zoom-Linse bewegt, wodurch die Brennweite der Fokussieroptik verändert wird. Um den Fokus in der Tiefe mit der erforderlichen Genauigkeit positionieren zu können, muß die Zoom-Linse mit sehr hoher Genauigkeit verstellt werden, was eine entsprechend aufwendige Mechanik erfordert.

In US 6,751,033 B2 ist ein ophthalmologisches Lasersystem gezeigt, bei dem ein von einer Laserstrahlquelle abgegebener Laserstrahl in lateraler Richtung abgelenkt und danach unter Verwendung einer Optik mit variabler Brennweite in einer vorgegebenen Tiefe fokussiert wird. Bei diesem System sind die gleichen Nachteile zu erwarten wie bei dem System der US 2003/0053219 A1, auch die WO 2005/011547 offenbart ein solches Lasersystem. In der US 2004/0243112 ist ein weiteres solches Lasersystem mit einem fs-Laser, welcher einen Pulskompressor aufweist, beschrieben.

Der vorliegenden Erfindung liegt daher die Aufgabe zugrunde, eine Scanvorrichtung zur Fokussierung eines Strahlenbündels in vorgegebene Bereiche eines vorgegebenen Volumens zu schaffen, mittels derer ein Laserstrahl einfach und genau in lateraler Richtung und in Richtung des Laserstrahls in verschiedene vorgegebene Bereiche eines Volumens fokussierbar ist.

Die Aufgabe wird gelöst durch die Merkmale von Anspruch 1 sowie die Merkmale von Anspruch 3, vorteilhafte Ausgestaltungen sind in den Unteransprüchen angeführt.

Das erfindungsgemäße Lasersystem weist dazu eine Scanvorrichtung zur Fokussierung eines Strahlenbündels in vorgegebene Bereiche eines vorgegebenen Volumens mit einer Eingangsoptik auf, in die das Strahlenbündel zuerst eintritt und die wenigstens ein erstes optisches Element aufweist, einer Fokussieroptik, mittels derer das aus der Eingangsoptik ausgetretene Strahlenbündel fokussierbar ist, und einer zwischen dem ersten optischen Element und der Fokussieroptik angeordnete Ablenkeinrichtung zur Ablenkung des Strahlenbündels nach passieren des ersten optischen Elements entsprechend einer einzustellenden Lage des Fokus in lateraler Richtung, wobei mit passieren hindurchtreten oder reflektieren gemeint ist, und wobei zur Einstellung der Lage des Fokus des Strahlenbündels in Richtung des Strahlenbündels wenigstens ein optisches Element der Eingangsoptik relativ zu der Ablenkeinrichtung bewegbar ist.

Die Scanvorrichtung verfügt also in Richtung des Strahlenbündels, das von einer Quelle für optische Strahlung, beispielsweise einem Laser, abgegeben werden kann, über die Eingangsoptik, dieser nachgeordnet die Ablenkeinrichtung und schließlich die Fokussieroptik. Eingangsoptik und Fokussieroptik wirken zusammen, um die Lage des Fokus des Strahlenbündels in Richtung des Strahlenbündels verstellen zu können, wozu ein optisches Element der Eingangsoptik, das insbesondere auch das erste optische Element der Eingangsoptik sein kann, in Abhängigkeit von der gewünschten Lage des Fokus in Richtung des Strahlenbündels relativ zu der Ablenkeinrichtung bzw. der Fokussieroptik bewegt wird. Eine Bewegung des Fokus in lateraler Richtung, das heißt quer zu der Richtung des Strahlenbündels, erfolgt durch die Ablenkeinrichtung, die durch Steuersignale, die dieser beispielsweise von einer geeigneten Steuervorrichtung zugeführt werden, derart ansteuerbar ist, daß der Fokus in lateraler Richtung in eine gewünschte bzw. durch die Steuersignale gegebene Lage bewegt werden kann.

Durch die Anordnung der Ablenkeinrichtung zwischen dem ersten optischen Element, das vorzugsweise, aber nicht notwendigerweise, das in Strahlrichtung erste Element der Eingangsoptik sein kann, und der Fokussieroptik werden zum einen die Anforderungen an das gesamte optische System herabgesetzt, da die Eingangsoptik nur für achsnahe Bündel ausgelegt zu werden braucht. Das vereinfacht die Korrektion und reduziert die Ausdehnung der Elemente. Zum anderen kann, da die Fokussierung durch Bewegung eines optischen Elements der Eingangsoptik erfolgt, die Fokussieroptik einfacher ausgelegt werden.

Insgesamt kann dadurch eine schnelle und einfache Einstellung der Lage des Fokus in drei Dimensionen in dem vorgegebenen Volumen erzielt werden.

Vorzugsweise ist durch Bewegung des relativ zu der Ablenkeinrichtung bewegbaren optischen Elements relativ zu der Ablenkeinrichtung die Divergenz des aus der Eingangsoptik austretenden Strahlenbündels veränderbar. Eine solche Ausführung der Eingangsoptik hat den Vorteil, daß die Eingangsoptik sehr einfach aufgebaut sein kann. Die Fokussieroptik fokussiert dann das aus der Eingangsoptik ausgetretene Strahlenbündel in Abhängigkeit von dessen Divergenzwinkel in verschiedene Tiefen, das heißt Lagen in Richtung des Strahlenbündels, ohne daß eine Bewegung der Fokussieroptik notwendig wäre. Die Aufgabe wird daher weiterhin auch gelöst durch ein Verfahren zur Fokussierung eines Strahlenbündels in vorgegebene Bereiche eines Volumens, bei dem in Abhängigkeit von der Lage des jeweiligen Bereichs die Divergenz des Strahlenbündels geändert, das Strahlenbündel quer zu seiner Ausbreitungsrichtung abgelenkt und dann fokussiert wird. Unter einer Veränderung der Divergenz des Strahlenbündels wird dabei verstanden, daß sich der Divergenz- bzw. Konvergenzwinkel des Strahlenbündels ändert. Das der Scanvorrichtung zugeführte Strahlenbündel kann dabei konvergent, divergent oder vorzugsweise parallel sein. Nach der Änderung der Divergenz und vor der Ablenkeinrichtung kann es dann in Abhängigkeit von der gewünschten Lage des Fokus divergent, parallel oder konvergent sein. Besonders bevorzugt sind Eingangsoptik und Fokussieroptik so ausgelegt und relativ zueinander angeordnet, daß ein paralleles Strahlenbündel von der Eingangsoptik wiederum in ein paralleles Strahlenbündel überführt wird, wenn der Fokus näherungsweise in der Mitte des Tiefenbereichs in dem vorgegeben Volumen liegt. Dies hat den Vorteil, daß eine Korrektion des Systems möglich ist, bei der zwischen den Abbildungsfehlern bei geringer Eindringtiefe des Fokus in dem Volumen und den Abbildungsfehlern bei großer Eindringtiefe vermittelt wird.

Das erste optische Element der Eingangsoptik kann beispielsweise durch eine Linse oder ein diffraktiv-optisches Element gegeben sein.

Das optische Element, das relativ zu der Ablenkeinrichtung bewegbar ist, kann beispielsweise eine Linse sein. Bei einer anderen bevorzugten Ausführungsform ist das relativ zu der Ablenkeinrichtung bewegbare optische Element ein diffraktiv-optisches Element.

Grundsätzlich kann die Eingangsoptik neben dem ersten optischen Element noch weitere optische Elemente aufweisen. Vorzugsweise umfaßt die Eingangsoptik eine Linse oder Linsengruppe negativer Brechkraft und eine dieser in Richtung des Strahlenbündels nachgeordnete Linse oder Linsengruppe positiver Brechkraft. Eine solche Anordnung zeichnet sich in vorteilhafter Weise durch einen besonders kompakten Aufbau aus und weist im Gegensatz zu einem Expander mit zwei Elementen positiver Brechkraft kein reelles Zwischenbild auf. Das erste optische Element der Eingangsoptik kann dann insbesondere durch die Linse negativer Brechkraft oder die erste Linse der Linsengruppe negativer Brechkraft gegeben sein. Die Linsen oder Linsengruppen können dabei insbesondere so angeordnet sein, daß die Eingangsoptik wenigstens für eine Stellung des relativ zu der Ablenkeinrichtung bewegbaren optischen Elements einen Expander darstellt, mittels dessen ein Querschnitt eines parallelen, einfallenden Strahlenbündels verändert, insbesondere vergrößert werden kann. Eine solche Anordnung zeichnet sich in vorteilhafter Weise durch einen besonders kompakten Aufbau aus.

Bei anderen Ausführungsformen können statt der Linse oder Linsengruppe negativer Brechkraft und/oder der Linse oder Linsengruppe positiver Brechkraft ein entsprechend wirkendes diffraktiv-optisches Element (DOE) bzw. ein Gruppe diffraktiv-optischer Elemente oder ein entsprechend wirkendes reflektives optisches Element bzw. eine Gruppe reflektiv optischer Elemente eingesetzt werden. Die Linsen oder Linsengruppen können also teilweise oder vollständig durch diffraktiv-optische Element oder Spiegel ersetzt werden. Die gewünschte Funktion der Änderung der Divergenz wird dann mit anderen Elementen erzielt. So kann in einer Ausgestaltung eine Eingangsoptik mit einem in Strahlrichtung ersten diffraktiv-optischen Element und einem dem ersten in Strahlrichtung nachgeordneten zweiten diffraktiv-optischen Element verwendet werden. In einer Grundstellung überführt das erste diffraktiv-optische Element ein paralleles Bündel in ein konvergentes Bündel und das zweite diffraktiv-optische Element überführt das konvergente Bündel wieder in ein paralleles Bündel, wobei sich durch entsprechende Ausbildung und Anordnung der diffraktiv-optischen Elemente eine Aufweitung des Bündels ergibt. Zur Änderung der Divergenz des aus der Eingangsoptik austretenden Bündels wird der Abstand zwischen dem ersten und dem zweiten diffraktiv-optischen Element geändert. Als weitere Ausgestaltung sei eine Eingangsoptik mit zwei Hohlspiegeln genannt. Der im Strahlengang erste Hohlspiegel erzeugt ein reelles Zwischenbild, das von dem dem ersten Hohlspiegel im Strahlengang nachgeordneten zweiten Hohlspiegel nach Unendlich abgebildet wird. Durch Abstandsänderung zwischen den beiden Hohlspiegeln wird die Divergenz gesteuert. Das Brennweitenverhältnis bestimmt das Verhältnis zwischen dem Durchmesser des austretenden Bündels und dem Durchmesser des eintretenden Bündels. Es können auch Kombinationen von Linsen, diffraktiv-optischen Elementen und Spiegeln verwendet werden.

Vorzugsweise ist das relativ zu der Ablenkeinrichtung bewegbare optische Element die Linse negativer Brechkraft oder eine Linse der Linsengruppe negativer Brechkraft. In diesem Fall braucht die Eingangsoptik in vorteilhafter Weise nur über eine besonders geringe Anzahl von Komponenten zu verfügen. Insbesondere kann das erste optische Element der Eingangsoptik das relativ zu der Ablenkeinrichtung bewegbare optische Element sein. Dieser Aufbau der Eingangsoptik ermöglicht es zum einen, die Linse negativer Brechkraft und damit das bewegbare optische Element sehr klein auszulegen, was zur Folge hat, daß es aufgrund der dann nur geringen Masse sehr schnell bewegbar ist. Zum anderen ergibt sich ein besonders günstiges Übersetzungsverhältnis zwischen der Bewegung der Linse negativer Brechkraft und der entsprechenden Bewegung des Fokus in Richtung des Strahlenbündels, das heißt, daß bei entsprechender Auslegung der Eingangsoptik und der Fokussieroptik eine Bewegung der Linse negativer Brechkraft um eine vorgegebene Strecke eine wesentlich geringere entsprechende Bewegung des Fokus in der Tiefe zur Folge hat, so daß die Anforderungen an die Genauigkeit eines Antriebs für die Linse negativer Brechkraft zur Erzielung einer vorgegebenen Genauigkeit der Einstellung der Lage des Fokus in der Tiefe relativ gering sind.

Um eine kontrollierte Bewegung des relativ zu der Ablenkeinrichtung bewegbaren optischen Elements zu ermöglichen, ist dieses vorzugsweise in einer, insbesondere parallel zum Strahlengang, geführt bewegbaren Halterung gehalten.

Vorzugsweise umfaßt die Scanvorrichtung weiterhin eine Antriebseinrichtung zur Bewegung des relativ zu der Ablenkeinrichtung bewegbaren optischen Elements. Diese kann, neben einer entsprechenden Lagerung des bewegbaren optischen Elements, noch einen Aktor aufweisen, der Steuersignale in eine entsprechende Bewegung umsetzt. Beispielsweise kann als Aktor ein Piezoaktor, ein elektrischer Linear-Motor oder auch einen Elektromotor, dessen Drehbewegung über ein Getriebe, beispielsweise einen Exzenterantrieb, in eine lineare Bewegung umgesetzt wird, eingesetzt werden. Bei Verwendung von Elektromotoren werden vorzugsweise Schrittmotoren verwendet, was den Vorteil hat, daß deren Bewegung auch ohne Regelung genau steuerbar ist. Andernfalls kann die Antriebseinrichtung über einen Regelkreis verfügen, der neben dem Aktor einen Sensor zur Erfassung der Lage des relativ zu der Ablenkeinrichtung bewegbaren optischen Elements und eine Regelschaltung umfaßt, die den Aktor in Abhängigkeit von der von dem Sensor erfaßten Lage des optischen Elements so ansteuert, daß dieses in eine vorgegebene Soll-Lage bewegt wird.

Die Ablenkeinrichtung kann grundsätzlich an einer beliebigen Position zwischen dem ersten optischen Element der Eingangsoptik und der Fokussieroptik angeordnet sein. Es ist jedoch bevorzugt, daß die Ablenkeinrichtung zwischen der Eingangsoptik und der Fokussieroptik angeordnet ist. Diese Anordnung hat den Vorteil, daß die Eingangsoptik einfach aufgebaut sein kann, da das Strahlenbündel dann in der Eingangsoptik nicht in lateraler Richtung bewegt wird, wenn das Strahlenbündel durch die Ablenkeinrichtung in lateraler Richtung bewegt wird.

Die Ablenkeinrichtung kann grundsätzlich in bekannter Art und Weise aufgebaut sein. Beispielsweise kann sie über ein für Strahlung des Strahlenbündels reflektives Element, zum Beispiel einen Spiegel, verfügen, der um zwei voneinander verschiedene, vorzugsweise zueinander orthogonale, Achsen kippbar ist, wozu die Ablenkeinrichtung weiterhin über wenigstens einen Aktor verfügt, mittels dessen das reflektive Element um die beiden Achsen in Abhängigkeit von Steuersignalen kippbar ist. Diese Ausführungsform hat den Vorteil, daß nur ein reflektives Element benötigt wird.

Vorzugsweise weist die Ablenkeinrichtung jedoch zwei voneinander beabstandete relativ zueinander und zu der Fokussieroptik bewegbare reflektive Elemente auf. Ein solcher Aufbau erlaubt in vorteilhafter Weise mit einfachen Mitteln eine sehr genaue Lagerung und Einstellung der reflektiven Elemente und damit der Lage des Fokus in lateraler Richtung, d.h. in einer Richtung quer zum Strahlenbündel nahe dessen Fokus. Die Ablenkeinrichtung kann in diesem Fall weiterhin über zwei Aktoren verfügen, die jeweils Steuersignale entsprechend einer gewünschten lateralen Ablenkung bzw. Lage des Fokus in eine Bewegung des jeweiligen reflektiven Elements umsetzt. Besonderes bevorzugt sind die für Strahlung des Strahlenbündels reflektiven Elemente um Achsen kippbar, die zueinander orthogonal sind. Wenigstens eines der reflektiven Elemente kann insbesondere als Spiegel ausgebildet sein.

Zwischen den reflektiven Elementen ist dann vorzugsweise eine Pupillenoptik angeordnet, die das erste reflektive Element auf das zweite reflektive Element abbildet. Diese Anordnung hat den Vorteil, daß sich eine eindeutige Pupillenlage ergibt, was insbesondere dann der Fall ist, wenn das reflektive Element, auf das das Strahlenbündel zuerst trifft, als Pupille wirkt. Eine eindeutige und feste Lage der Pupille ist bei anspruchsvollen, d.h. sehr präzise arbeitenden, optischen Systemen eine Voraussetzung für eine gute Korrektion.

Zur Verstellung des Fokus in der Tiefe, d.h. in Richtung des Strahlenbündels, wird erfindungsgemäß das relativ zu der Ablenkeinrichtung bewegbare optische Element bewegt. Dies schließt nicht aus, daß die Fokussieroptik eine veränderbare Brennweite hat. Es ist jedoch bevorzugt, daß die Fokussieroptik eine feste Brennweite aufweist. Insbesondere umfaßt diese dann keine Zoom-Linse. Bei dem erfindungsgemäßen Verfahren ist es bevorzugt, daß zur Fokussierung des abgelenkten Strahlenbündels eine Fokussieroptik fester Brennweite verwendet wird. Die Fokussieroptik ist weiter vorzugsweise relativ zu der Ablenkeinrichtung fest angeordnet. Bei diesen Ausführungsformen erfolgt die Verstellung der Lage des Fokus also nicht über eine Veränderung der Fokussieroptik, was den Vorteil hat, daß diese besonders einfach ausgelegt werden kann.

Vorzugsweise ist in dem Strahlengang zwischen der Ablenkeinrichtung und wenigstens einer Austrittslinse oder Austrittslinsengruppe der Fokussieroptik ein Strahlteiler angeordnet. Unter einer Austrittslinse oder Austrittslinsengruppe der Fokussieroptik wird dabei die Linse oder Linsengruppe der Fokussieroptik verstanden, aus der das Strahlenbündel in das Volumen, in das es fokussiert werden soll, austritt. Der Strahlteiler ist dabei vorzugsweise so angeordnet, daß einerseits das aus der Ablenkeinrichtung ausgetretene, abgelenkte Strahlenbündel in die Austrittslinse oder Austrittslinsengruppe fällt, und andererseits wenigstens ein Teil eines aus dem Volumen kommenden Beobachtungsstrahlenbündels aus dem Strahlengang zwischen der Ablenkeinrichtung und der Austrittslinse oder Austrittslinsengruppe ausgekoppelt wird. Je nach Wellenlänge der Strahlung des von der Scanvorrichtung zu fokussierenden Strahlenbündels kann der Strahlteiler insbesondere dichroitisch ausgelegt sein, so daß er das Strahlenbündel und das Beobachtungsstrahlenbündel unterschiedlich beeinflußt. Eine solche Scanvorrichtung hat den Vorteil, daß das Volumen während der Einstrahlung des Strahlenbündels unter Verwendung wenigstens des Teils des aus dem Volumen kommenden Beobachtungsstrahlenbündels einfach beobachtet werden kann. Insbesondere in dem Fall, daß die Fokussieroptik eine feste Brennweite aufweist und relativ zu der Ablenkeinrichtung fest angeordnet ist, kann das Volumen unabhängig von der Lage des Fokus des Strahlenbündels mit einer fest eingestellten Optik oder Kamera beobachtet werden.

Vorzugsweise ist die Austrittslinse oder die Austrittslinsengruppe so ausgebildet, daß ein paralleles Strahlenbündel in das vorgegebene Volumen fokussiert wird. Die Austrittslinse oder Austrittslinsengruppe kann dann als Objektivlinse oder Objektivlinsengruppe aufgefaßt werden. Vorzugsweise ist dabei die Austrittslinse oder Austrittslinsengruppe so ausgelegt, daß das gesamte Volumen im wesentlichen scharf nach Unendlich abgebildet wird, so daß für die Mitbeobachtung die Tiefenschärfe so groß ist, daß in vorteilhafter Weise das ganze Volumen ohne Verstellung der Beobachtungsoptik scharf abgebildet und beobachtet werden kann.

Die Fokussieroptik kann in Teiloptiken zerlegt sein. Vorzugsweise weist die Fokussieroptik ein Eintrittsobjektiv zur Erzeugung eines reellen Zwischenbildes einer das Strahlenbündel abgebenden Strahlungsquelle auf. Die Fokussieroptik kann dann vorzugsweise noch eine Tubusoptik aufweisen, die das Zwischenbild nach Unendlich abbildet. Das von der Tubusoptik nach Unendlich abgebildete Zwischenbild wird dann von der Austrittslinse oder -linsengruppe bzw. der Austrittsoptik in das Zielvolumen fokussiert. Durch diese mehrteilige Auslegung kann die Fokussieroptik wesentlich einfacher korrigiert werden. So können Kompensationseffekte zwischen den verschiedenen Teilen der Fokussieroptik, beispielsweise des Eintrittsobjektivs und der Tubusoptik ausgenutzt werden. Die Teiloptiken können größere Brennweiten haben als die Fokussieroptik insgesamt. Dadurch kann beispielsweise das Verhältnis zwischen dem Durchmesser des eintretenden Bündels und der Brennweite des Eintrittsobjektivs günstig gestaltet werden, was die Korrektion erleichtert. Darüber hinaus entsteht durch den parallelen Strahlengang zwischen der Tubusoptik und der Austrittsoptik eine mögliche Schnittstelle für die Mitbeobachtung. Insbesondere kann die Brennweite der Austrittslinse oder Austrittslinsengruppe so gewählt werden, daß eine Mitbeobachtung einfach möglich ist.

Die Scanvorrichtung kann für beliebige optische Strahlungsquellen verwendet werden, vorzugsweise jedoch für Laser. Insbesondere in diesem Fall ist es bevorzugt, das alle reellen Zwischenbilder einer das Strahlenbündel abgebenden Strahlungsquelle in Gas, insbesondere Luft oder Vakuum, liegen. Dies hat den Vorteil, daß bei der Verwendung der Scanvorrichtung für Laserstrahlung sehr hoher Intensität keine optischen Durchbrüche in den optischen Komponenten der Scanvorrichtung, beispielsweise Linsen oder Spiegeln, auftreten können, die diese beschädigen könnten.

Erfindungsgemäß sind die Eingangs- und die Fokussieroptik über die spektrale Breite vorgegebener Femtosekundenpulse so chromatisch korrigiert, daß durch sie hindurchtretende Femtosekundenpulse mit einer dispersionsbedingten zeitlichen Verbreiterung von weniger als 30 % fokussiert werden können. Vorzugsweise ist das gesamte optische System der Scanvorrichtung entsprechend korrigiert. Durch ein angepasstes Dispersionsmanagement vor dem Systemeintritt des Pulses und die schon erwähnte chromatische Korrektion des gesamten optischen Systems kann im Fokus eine Pulsdauer erzielt werden, die weniger als 30% über der theoretisch erreichbaren Pulsdauer liegt. Vorzugsweise ist diese chromatische Korrektur für Femtosekundenpulse mit Wellenlängen in einem Bereich um 1040 nm ausgelegt. Eine solche Scanvorrichtung eignet sich daher insbesondere für laserchirurgische Systeme, die zur Bildung eines Corneadeckels bei einer LASIK-Behandlung unter Verwendung von Femtosekundenpulsen gedacht sind.

Darüber hinaus ist das optische System vorzugsweise beugungsbegrenzt korrigiert, so daß beispielsweise in lateraler Richtung durch die Optik verursachte Abbildungsfehler weitgehend unterdrückt werden bzw. auf ein Minimum reduziert sind.

Die Fokussieroptik verfügt weiterhin vorzugsweise über eine Appretur von wenigstens 0,35.

Insbesondere bei Verwendung der Scanvorrichtung für laserchirurgische Zwecke ist es bevorzugt, daß das relativ zu der Ablenkeinrichtung bewegliche optische Element über eine so große Strecke relativ zu der Ablenkeinrichtung bewegbar ist, das der Fokus des Strahlenbündels in Strahlrichtung in einem Bereich größer als 0,5 mm bewegbar ist. Eine solche Ausbildung hat den Vorteil, daß bei Verwendung der Scanvorrichtung für eine Behandlung mit LASIK ohne Bewegung der Cornea relativ zu der Scanvorrichtung ein Corneadeckel einfach gebildet werden kann.

Zu dem gleichen Zweck ist es bevorzugt, daß die Ablenkeinrichtung so ausgebildet ist, daß der Fokus des Strahlenbündels in dem vorgegebenen Volumen in einem Bereich in einer lateralen Ebene bewegbar ist, der einen Durchmesser von 11 mm hat. Dies hat den Vorteil, daß wiederum ohne Bewegung der Cornea relativ zu der Scanvorrichtung ein bei der Behandlung mit LASIK notwendiger Corneadeckel ohne Bewegung der Cornea gebildet werden kann.

Die Scanvorrichtung kann mit beliebigen Quellen für optische Strahlung verwendet werden, weist vorzugsweise jedoch einen Femtosekundenlaser zur Abgabe von Femtosekundenpulsen entlang des Strahlenbündels auf, wobei der Laser, die Eingangsoptik und die Fokussieroptik so ausgelegt sind, daß das fokussierte Strahlenbündel im Fokus einen Durchmesser kleiner als 5 µm hat. Eine solche Scanvorrichtung eignet sich insbesondere zur sehr präzisen Präparation des Corneadeckels bei einer LASIK-Behandlung.

Die Scanvorrichtung kann jedoch auch zu anderen Zwecken verwendet werden.

Die Erfindung wird im folgenden noch näher unter Verwendung der Zeichnungen erläutert. Es zeigen:
- Fig.1: eine schematische und vereinfachte Darstellung eines Systems zur laserchirurgischen Behandlung des Auges mit einer Scanvorrichtung nach einer ersten bevorzugten Ausführungsform der Erfindung,
- Fig.2: eine schematische teilweise Darstellung eines laserchirurgischen Behandlungssystems nach einer zweiten bevorzugten Ausführungsform der Erfindung,
- Fig.3: eine schematische Darstellung eines Systems zur laserchirurgischen Behandlung des Auges mit einer Scanvorrichtung nach einer dritten bevorzugten Ausführungsform der Erfindung.

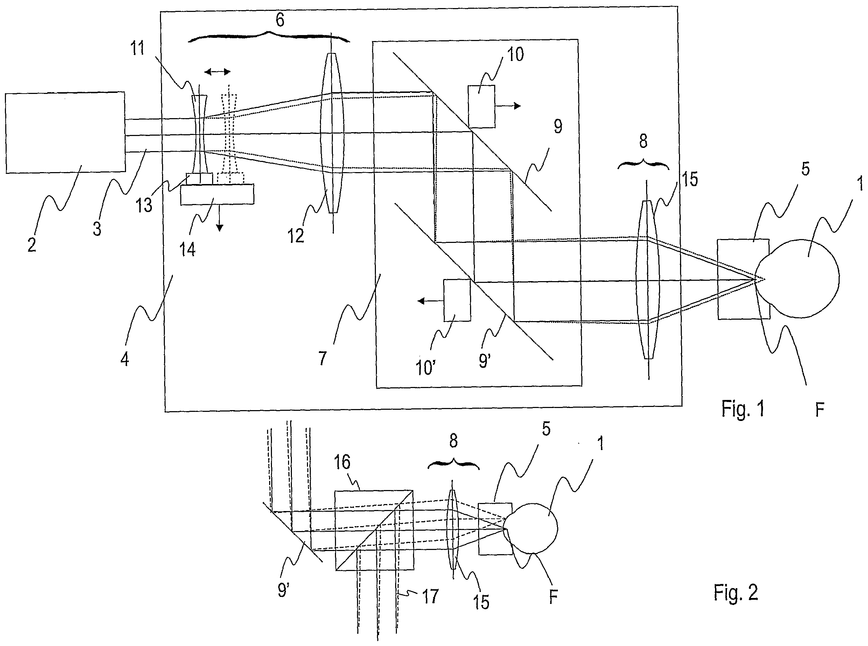

In Fig.1 umfaßt ein laserchirurgisches System zur Behandlung eines menschlichen Auges 1 eine Strahlungsquelle 2 in Form eines Femtosekundenlasers zur Abgabe von gepulster Laserstrahlung in Form eines gepulsten Strahlenbündels 3 und eine Scanvorrichtung 4 nach einer ersten bevorzugten Ausführungsform der Erfindung, mittels derer das Strahlenbündel 3 in verschiedene, vorgegebene Bereiche in einem dreidimensionalen Volumen in der Cornea des Auges 1 fokussierbar ist. Auf der Cornea des Auges 1 befindet sich ein Kontaktglas 5 mit einer konkaven Kontaktfläche, an der die Cornea des Auges 1 anliegt, wodurch eine Bewegung der Cornea während einer Behandlung mit der Laserstrahlung möglichst weitgehend unterdrückt werden soll.

Die Strahlungsquelle 2 in Form eines Femtosekundenlasers ist zur Abgabe von Femtosekundenpulsen im Wellenlängenbereich um 1040 nm mit einer Pulsbreite im Bereich von etwa 200 fs ausgebildet und an sich bekannt. Sie kann insbesondere neben dem eigentlichen Laser noch pulsformende Einrichtungen umfassen.

Die Scanvorrichtung 4 verfügt in Richtung des vom Laser ausgehenden Strahlenbündels 3 über eine Eingangsoptik 6, durch die das Strahlenbündel 3 in die Scanvorrichtung 4 eintritt, eine Ablenkeinrichtung 7, die das aus der Eingangsoptik 6 austretende Strahlenbündel 3 entsprechend vorgegebener Steuersignale in lateraler Richtung, das heißt quer zur Richtung des in die Ablenkeinrichtung 7 einfallenden Strahlenbündels ablenkt, und eine relativ zu der Ablenkeinrichtung 7 fest angeordnete Fokussieroptik 8 zur Fokussierung des aus der Eingangsoptik 6 ausgetretenen und von der Ablenkeinrichtung 7 abgelenkten Strahlenbündels 3, die dieses in den Bereich der Cornea des Auges 1 fokussiert.

Die nur schematisch gezeigte Ablenkeinrichtung 7 ist in an sich bekannter Weise ausgebildet und verfügt über zwei für die Strahlung des Strahlungsbündels 3 reflektive Elemente 9 und 9' in Form von Spiegeln, die um in Fig.1 nicht gezeigte Achsen kippbar gelagert sind. Zur einfacheren Darstellung sind in Fig.1 die reflektiven Elemente 9 und 9' in nur schematischer Weise parallel zueinander dargestellt, tatsächlich jedoch verlaufen die Kippachsen orthogonal zueinander und zu einer optischen Achse der Eingangsoptik 6, so daß durch Kippen des erste reflektiven Elements 9 das Strahlenbündel 3 in y-Richtung, d. h. in Fig.1 nach oben oder unten, und durch Kippen des zweiten reflektiven Elements 9' in einer zu der y-Richtung und der optischen Achse der Eingangsoptik orthogonalen x-Richtung, in Fig.1 in bzw. aus der Zeichenebene, abgelenkt werden kann. Die reflektiven Elemente bzw. Spiegel 9 und 9' sind galvanometrisch ausgelenkt, wozu Aktoren 10 bzw. 10' vorgesehen sind, die über in Fig.1 durch Pfeile angedeutete Signalverbindungen mit einer Steuereinrichtung verbunden sind. Die Steuereinrichtung gibt entsprechend der gewünschten Lage des Fokus des Strahlenbündels 3 in lateraler Richtung, d.h. in x- und y-Richtung, Steuersignale an die Aktoren 10 und 10' ab, die daraufhin die reflektiven Elemente bzw. Spiegel 9 und 9' in bekannter Weise kippen.

Die Eingangsoptik 6 verfügt über ein relativ zu der Ablenkeinrichtung 7 bewegbares optisches Element in Form einer ersten Linse 11 negativer Brechkraft, d.h. eine Zerstreuungslinse, als erstes optisches Element sowie eine Sammellinse 12, d.h. eine Linse positiver Brechkraft. Die optischen Achsen der beiden Linsen verlaufen koaxial. Die erste Linse 11 ist in einem Linsenhalter 13 gehalten, der in einer in Fig.1 nicht gezeigten Führung linear in einer Richtung parallel zur optischen Achse der ersten Linse 11 verschiebbar ist, so daß die erste Linse 11 als optisches Element der Eingangsoptik 6 relativ zu der Ablenkeinrichtung 7 bewegbar ist. Zur Bewegung des Linsenhalters 13 und damit auch der ersten Linse 11 parallel zu deren optischer Achse dient ein in Fig.1 nur schematisch gezeigter Aktor, im Beispiel ein Linearantrieb 14, der, wiederum angedeutet durch einen Pfeil, mit der nicht gezeigten Steuereinrichtung verbunden ist. Diese sendet zur Bewegung der ersten Linse 11 in Abhängigkeit von der gewünschten Lage des Fokus entlang der Richtung des Strahlenbündels 3 im Bereich des Fokus Steuersignale an den Linearantrieb 14. Im Beispiel ist der Linearantrieb 14 durch einen Schritt-Linear-Motor realisiert.

In einer in Fig.1 durch durchgezogene Linien veranschaulichten Stellung des relativ zu der Ablenkeinrichtung 7 bewegbaren optischen Elements bzw. der ersten Linse 11 zu der Sammellinse 12 wirkt die Eingangsoptik 6 als Expander, der den Querschnitt des Strahlenbündels 3 aufweitet, wobei bei einem vor der Eingangsoptik 6 parallelen Strahlenbündel 3 das aus dieser austretende Strahlenbündel 3 wiederum parallel ist.

Die Fokussieroptik 8 ist in Fig.1 in diesem Ausführungsbeispiel nur als eine relativ zu der Ablenkeinrichtung 7 feststehende Austrittslinse 15 bzw. Objektivlinse in Form einer Sammellinse dargestellt, die das aus der Eingangsoptik 6 ausgetretene und durch die Ablenkeinrichtung 7 in lateraler Richtung abgelenkte Strahlenbündel 3 in den Bereich der Cornea des Auges 1 fokussiert. Die Austrittslinse 15 ist jedoch nur eine schematische Darstellung eines optischen Systems positiver Brechkraft, das komplexer aufgebaut ist, wobei der Aufbau im Rahmen dieser Darstellung keine Rolle spielt.

Die Lage des Fokus F des Strahlenbündels 3 in der Richtung entlang des Strahlenbündels wird durch die Eingangsoptik 6 und die Fokussieroptik 8 bestimmt. Die Lage des Fokus F in der Richtung des Strahlenbündels wird durch Verfahren der ersten Linse 11 entlang ihrer optischen Achse relativ zu der Ablenkeinrichtung 7 und der Fokussieroptik 8 eingestellt. Bei einer Änderung des Abstands zwischen der ersten Linse 11 und der Sammellinse 12 wird die Divergenz des vor der Linse 11 parallelen Strahlenbündels 3 verändert, was in Fig.1 beispielhaft durch gestrichelte Linien dargestellt ist. Je nach Stellung der ersten Linse 11 kann das aus der Sammellinse 12 austretende Strahlenbündel divergent, parallel oder konvergent sein. Je nach Divergenz des Strahlenbündels fokussiert die feststehende Fokussieroptik 8 dann das Strahlenbündel in unterschiedliche Abstände von der Fokussieroptik 8 bzw. der Austrittslinse 15.

In dem vorliegenden Beispiel kann die erste Linse 11 sehr einfach und schnell bewegt werden, da sie bei einem Durchmesser von ca. 6 mm klein und leicht ist. Darüber hinaus beträgt das Übersetzungsverhältnis der Bewegung des relativ zu der Ablenkeinrichtung 7 bewegbaren optischen Elements, d.h. der ersten Linse 11, in eine entsprechende Bewegung des Fokus F in Richtung des Strahlenbündels 3 in diesem Beispiel etwa 20:1, was wesentlich größer ist als bei Verwendung einer der Ablenkeinrichtung nachgeordneten Zoom-Optik, bei der typischerweise ein deutlich kleineres Übersetzungsverhältnis zu erwarten ist. In dem Beispiel in US 2003/0053219 A1 liegt es bei 1:1.

Fig.2 zeigt einen Ausschnitt aus einem laserchirurgischen System mit einer Scanvorrichtung nach einer zweiten bevorzugten Ausführungsform der Erfindung. Die Scanvorrichtung unterscheidet sich von der ersten Scanvorrichtung 4 dadurch, daß nun im Strahlengang zwischen der Ablenkeinrichtung 7 und der Fokussieroptik 8 mit der als Austrittslinse 15 wirkenden Sammellinse ein Strahlteiler 16 angeordnet ist, mittels dessen von Punkten in der Cornea des Auges 1 ausgehende Beobachtungsstrahlenbündel 17, die bei Abbildung von Objektpunkten in der Cornea des Auges 1 durch die Austrittslinse 15 näherungsweise ins Unendliche entstehen, wenigstens teilweise aus dem Strahlengang zwischen der Ablenkeinrichtung 7 und der Fokussieroptik 8 in Richtung auf einen in den Figuren nicht gezeigten Tubus mit Okular oder ein in den Figuren nicht gezeigtes Objektiv mit Kamera abgelenkt werden können, so daß eine Mitbeobachtung der Cornea während der Abgabe des Strahlenbündels 3 auf die Cornea möglich ist. Dadurch, daß mindestens für die Behandlungsdauer die Fokussieroptik 8 und die Cornea des Auges 1 eine feste Relativlage zueinander einnehmen und der Brennpunkt der Fokussieroptik 8 in dem Bearbeitungsvolumen liegt, erfolgt die Mitbeobachtung mit konstanter Schärfe auch dann, wenn der Fokus des Strahlenbündels 3 in der Tiefe, d.h. in einer Richtung parallel zur Richtung des Strahlenbündels 3 in der Nähe des Brennpunktes, bewegt wird. Bei der Festlegung der Beobachtungsapertur wird vorzugsweise berücksichtigt, daß einerseits ein ausreichend helles Bild mit guter Auflösung und andererseits eine möglichst große Schärfentiefe erzielt werden kann.

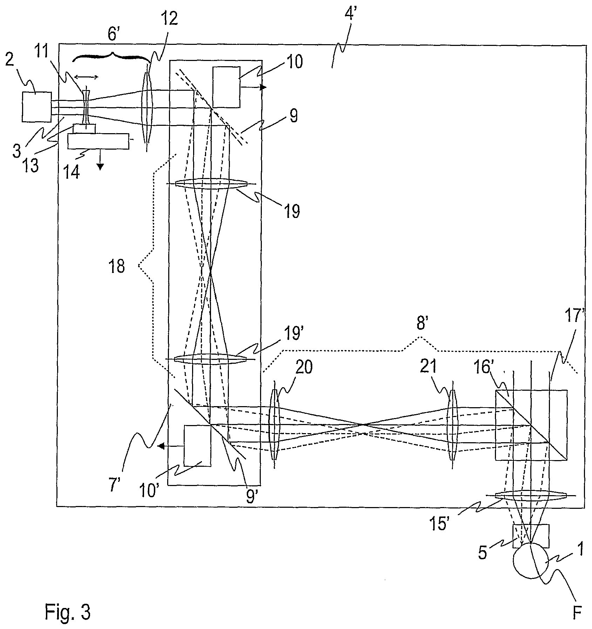

Fig.3 zeigt teilweise und schematisch ein laserchirurgisches System mit einer Scanvorrichtung 4' nach einer dritten bevorzugten Ausführungsform der Erfindung. Dabei sind Komponenten, die mit den Komponenten des ersten Ausführungsbeispiels übereinstimmen, mit den gleichen Bezugszeichen bezeichnet und die Ausführungen im ersten Ausführungsbeispiel zu diesen Komponenten gelten auch hier entsprechend.

Die Ablenkeinrichtung 7 des ersten Ausführungsbeispiels ist hier durch eine Ablenkeinrichtung 7' ersetzt, die sich von der Ablenkeinrichtung 7 nur durch den Abstand der reflektiven Elemente 9 und 9' voneinander unterscheidet. Die Kippachse des reflektiven Elements 9 verläuft orthogonal zu dem Strahlenbündel 3 und schneidet wenigstens ungefähr die optische Achse der Eingangsoptik 6. Entsprechendes gilt für das reflektive Element 9' und die Fokussieroptik 8'. In der Ruhelage sind die reflektiven Elemente 9 und 9' so zueinander ausgerichtet, daß ein entlang der optischen Achse der Eingangsoptik 6 einfallender Strahl im Bereich der Kippachse des zweiten reflektiven Elementes 9' auf diesen auftritt. In der Ruhelage sind die Spiegel gegenüber den optischen Achsen der Eingangsoptik 6 bzw. Fokussieroptik 8' in einem Winkel von etwa 45° geneigt.

Darüber hinaus ist in dem Strahlengang zwischen den reflektiven Elementen 9 und 9' eine Pupillenoptik 18 angeordnet, die zwei Sammellinsen 19 und 19' aufweist, die das von dem Strahlenbündel 3 zuerst getroffene reflektive Element 9 auf das andere reflektive Element 9' abbilden, wobei ein zwischen den Sammellinsen 19 und 19' entstehendes reelles Zwischenbild in Luft entsteht, um optische Durchbrüche in Komponenten der Scanvorrichtung zu vermeiden. Auf diese Weise ergibt sich eine feste Lage der Pupille, die eine günstige Auslegung der Fokussieroptik 8' erleichtert. Durch die Abbildung der reflektiven Elemente 9 und 9' ineinander kann darüber hinaus die Größe des reflektiven Elementes 9' klein gehalten werden, im Beispiel haben die Spiegel die Form einer Ellipse, deren Haupt- bzw. Nebenachse eine Länge von etwa 21 mm bzw. 15 mm aufweisen.

Die Fokussieroptik 8' unterscheidet sich von der Fokussieroptik 8 in mehrfacher Hinsicht. Sie ist mehrteilig aufgebaut und verfügt über ein Eintrittsobjektiv 20, in die das aus der Ablenkeinrichtung 7' austretende, lateral abgelenkte Strahlenbündel 3 eintritt und in einem reellen Zwischenbild, fokussiert wird, eine Tubuslinse 21, die das Zwischenbild je nach dessen Lage nach Unendlich abbildet, einen im Strahlengang der Tubuslinse 21 nachgeordneten Strahlteiler 16', der das Strahlenbündel 3 auf die Austrittslinse 15' ablenkt, und die Austrittslinse 15', die das Strahlenbündel 3, das an dieser Stelle parallel oder nur schwach konvergent oder schwach divergent ist, dann entsprechend ihrer festen Brennweite auf die Cornea des Auges 1 fokussiert und somit als Objektivlinse fungiert.

Die Verstellung der Lage des Fokus in lateraler Richtung als auch in Richtung des Strahlenbündels 3 nahe dem Fokus erfolgt wie im ersten Ausführungsbeispiel.

Der Strahlteiler 16' ist so ausgebildet, daß Beobachtungsstrahlenbündel 17', die durch Abbildung von Punkten in der Cornea des Auges 1 durch die Austrittslinse 15' nach Unendlich entstehen, durch den Strahlteiler 16' hindurchtreten und einem in Fig.3 nicht gezeigten Tubus mit Okular oder einem in Fig.3 nicht gezeigten Objektiv mit einer Kamera zugeführt werden können. Es wird so, wie im zweiten Ausführungsbeispiel eine Mitbeobachtung während einer Behandlung der Cornea mit dem Strahlenbündel 3 ermöglicht.

Die Zerlegung der Fokussieroptik in ein Eintrittsobjektiv 20, eine Tubuslinse 21 und eine Austrittslinse 15' bzw. ein Hauptobjektiv hat neben der Möglichkeit, eine Schnittstelle für die Mitbeobachtung zu ermöglichen, den Vorteil, daß kleine Bündeldurchmesser am Eingang der Fokussieroptik realisiert werden können. Beispielsweise kann der Bündeldurchmesser 15 mm betragen, so daß die reflektiven Elemente 9 und 9' mit einem Arbeitsdurchmesser von 15 mm verwendet werden können. Diese relativ kleine Größe der reflektiven Elemente 9 und 9' ist günstig zur Erzielung hoher Scangeschwindigkeiten. Darüber hinaus ermöglicht die Zerlegung der Fokussieroptik in Teilsysteme eine beugungsbegrenzte Korrektur der Fokussieroptik. Da die Bündel zwischen den Teilsystemen nicht korrigiert sein müssen, können Kompensationseffekte genutzt werden, die die Zahl der notwendigen Linsen deutlich reduzieren.

Zur Vermeidung unerwünschter Wechselwirkungen sind alle optischen Komponenten in diesem wie auch in den anderen Ausführungsbeispielen so ausgelegt, daß alle reellen Zwischenbilder in Luft liegen und so auch sehr intensive Laserstrahlung nicht zu optischen Durchbrüchen in optischen Komponenten führen kann.

In den bis hierher beschriebenen Ausführungsbeispielen ist jeweils das gesamte optische System der Scanvorrichtung, insbesondere die Eingangs- und die Fokussieroptik, für die spektrale Breite eines zu verwendenden fs-Pulses chromatisch korrigiert. Durch ein angepasstes Dispersionsmanagement vor dem Systemeintritt des Pulses und die schon erwähnte chromatische Korrektion des gesamten optischen Systems kann im Fokus eine Pulsdauer erzielt werden, die dispersionsbedingt, d.h. durch chromatische Aberrationen bedingt, weniger als 30% über der theoretisch erreichbaren Pulsdauer liegt.

Darüber hinaus ist das relativ zu der Ablenkeinrichtung bewegbare optische Element, d.h. die Linse 11 über eine so große Strecke relativ zu der Ablenkeinrichtung bewegbar, daß der Fokus des Strahlenbündels in Strahlrichtung in einen Bereich größer als 0,5 mm bewegbar ist. Weiterhin ist die Ablenkeinrichtung so ausgebildet, daß der Fokus des Strahlenbündels in einem Bereich in einer lateralen Ebene bewegbar ist, der einen Durchmesser von 11 mm hat.

Der Laser, die Eingangsoptik und die Fokussieroptik sind so ausgelegt sind, daß das fokussierte Strahlenbündel im Fokus einen Durchmesser kleiner als 5 Mikrometer hat.

Die Fokussieroptik hat in den Ausführungsbeispielen vorzugsweise eine Apertur größer als 0,35.

Die laserchirurgischen Systeme eignen sich daher insbesondere zur Bildung eines klappbaren Corneadeckels durch Photodisruption mittels Femtosekundenpulsen.

Ein weiteres Ausführungsbeispiel unterscheidet sich von dem zweiten Ausführungsbeispiel durch die Ausbildung der Eingangsoptik. Die Linsen bzw. Linsengruppen sind hier durch diffraktiv-optische Elemente ersetzt. Die Eingangsoptik umfaßt ein erstes und ein zweites diffraktiv-optisches Element. Das erste diffraktiv-optische Element ist so ausgebildet und angeordnet, daß es in einer Grundstellung ein paralleles Bündel in ein konvergentes Bündel überführt. Das dem ersten im Strahlengang nachgeordnete zweite diffraktiv-optische Element ist so ausgebildet und angeordnet, daß es das konvergente Bündel wieder in ein paralleles Bündel überführt. Es ergibt sich so insgesamt eine Aufweitung des Bündels. Zur Änderung der Divergenz des aus der Eingangsoptik austretenden Bündels wird der Abstand zwischen dem ersten und dem zweiten diffraktiv-optischen Element geändert.

In einem anderen Ausführungsbeispiel ist die Eingangsoptik des vorhergehenden Ausführungsbeispiels dahingehend geändert, daß die Eingangsoptik nun zwei Hohlspiegel besitzt, die so angeordnet sind, daß sich zwischen dem Laser und der Ablenkeinrichtung ein zweifach gefalteter, näherungsweise z-förmiger Strahlengang ergibt. Der erste Hohlspiegel erzeugt ein reelles Zwischenbild, das von dem dem ersten Hohlspiegel im Strahlengang in einem geeigneten Grundabstand nachgeordneten zweiten Hohlspiegel nach Unendlich abgebildet wird. Durch Abstandsänderung zwischen den Hohlspiegeln wird die Divergenz gesteuert. Das Brennweitenverhältnis bestimmt das Verhältnis zwischen dem Durchmesser bzw. Öffnungswinkel des aus der Eintrittsoptik austretenden Bündels und dem Durchmesser bzw. Öffnungswinkel des in die Eintrittsoptik eintretenden Bündels.

### Bezugszeichenliste

- 1: Auge
- 2: Strahlungsquelle
- 3, 3": Strahlungsbündel
- 4, 4', 4": Scanvorrichtung
- 5: Kontaktglas
- 6, 6": Eingangsoptik
- 7, 7', 7": Ablenkeinrichtung
- 8, 8', 8": Fokussieroptik
- 9, 9': reflektive Elemente
- 10, 10': Aktoren
- 11: erste Linse
- 12: Sammellinse
- 13: Linsenhalter
- 14: Linearantrieb
- 15, 15': Austrittslinse
- 16, 16': Strahlteiler
- 17, 17': Beobachtungsstrahlenbündel
- 18: Pupillenoptik
- 19,19': Sammellinsen
- 20: Eintrittsobjektiv
- 21: Tubuslinse
- 22: Strahlungsquelle
- 23: Strahlteiler
- 24: Probe
- 25: Sammeloptik
- 26: Pinhohle-Blende
- 27: Detektionseinrichtung

## Patentansprüche

1. Laserchirurgisches System mit einer Strahlungsquelle (2) in Form eines Femtosekundenlasers und einer Scanvorrichtung (4) zur Fokussierung eines Strahlenbündels (3, 3") in vorgegebene Bereiche der Cornea eines Auges (1) umfassend eine Eingangsoptik (6, 6"), in die das Strahlenbündel (3, 3'') zuerst eintritt und die wenigstens ein erstes optisches Element (11) aufweist, eine Fokussieroptik (8, 8', 8") mittels derer das aus der Eingangsoptik (6, 6") ausgetretene Strahlenbündel (3, 3") fokussierbar ist, und eine zwischen dem ersten optischen Element (11) und der Fokussieroptik (8, 8', 8") angeordnete Ablenkeinrichtung (7, 7', 7") zur Ablenkung des Strahlenbündels (3, 3") nach passieren des ersten optischen Elements (11) entsprechend einer einzustellenden Lage des Fokus in lateraler Richtung, wobei zur Einstellung der Lage des Fokus des Strahlenbündels (3, 3") in Richtung des Strahlenbündels wenigstens das eine optische Element (11) der Eingangsoptik (6, 6") relativ zu der Ablenkeinrichtung (7, 7', 7") bewegbar ist, wobei das System ein Kontaktglas (5) mit konkaver Kontaktfläche aufweist, welches geeignet ist mit der konkaven Kontaktfläche an der Cornea des Auges (1) anzuliegen,
wobei bei der Scanvorrichtung die optischen Elemente der Eingangs- und Fokussieroptik (6, 6", 8, 8', 8") über die spektrale Breite der von der Strahlungsquelle abgegebenen Femtosekundenpulse so chromatisch korrigiert sind, daß durch sie durchtretende fs-Pulse mit einer dispersionsbedingten zeitlichen Verbreiterung von weniger als 30 % durch die Fokussieroptik fokussiert werden und wobei
bei der Scanvorrichtung die Eingangsoptik (6, 6") als das erste optisches Element (11) eine Linse oder Linsengruppe negativer Brechkraft und die Eingangsoptik zudem eine dieser in Richtung des Strahlenbündels (3, 3") nachgeordnete Sammellinse (12) oder Linsengruppe positiver Brechkraft umfaßt.

2. Laserchirurgisches System mit einer Scanvorrichtung nach Anspruch 1, bei der durch Bewegung des relativ zu der Ablenkeinrichtung (7, 7', 7") bewegbaren ersten optischen Elements (11) relativ zu der Ablenkeinrichtung (7, 7', 7") die Divergenz des aus der Eingangsoptik (6, 6") austretenden Strahlungsbündels (3, 3") veränderbar ist.

3. Laserchirurgisches System mit einer Strahlungsquelle (2) in Form eines Femtosekundenlasers und einer Scanvorrichtung (4) zur Fokussierung eines Strahlenbündels (3, 3") in vorgegebene Bereiche der Cornea eines Auges (1) umfassend eine Eingangsoptik (6, 6"), in die das Strahlenbündel (3, 3") zuerst eintritt und die wenigstens ein erstes optisches Element (11) aufweist, eine Fokussieroptik (8, 8', 8") mittels derer das aus der Eingangsoptik (6, 6") ausgetretene Strahlenbündel (3, 3") fokussierbar ist, und eine zwischen dem ersten optischen Element (11) und der Fokussieroptik (8, 8', 8") angeordnete Ablenkeinrichtung (7, 7', 7'') zur Ablenkung des Strahlenbündels (3, 3") nach passieren des ersten optischen Elements (11) entsprechend einer einzustellenden Lage des Fokus in lateraler Richtung, wobei das erste optischen Element (11) der Eingangsoptik (6, 6") zur Einstellung der Lage des Fokus des Strahlenbündels (3, 3") in Richtung des Strahlenbündels relativ zu der Ablenkeinrichtung (7, 7', 7") bewegbar ist, wobei durch die Bewegung des relativ zu der Ablenkeinrichtung (7, 7', 7") bewegbaren ersten optischen Elements (11) relativ zu der Ablenkeinrichtung (7, 7', 7") die Divergenz des aus der Eingangsoptik (6, 6") austretenden Strahlenbündels (3, 3") veränderbar ist,
wobei das System ein Kontaktglas (5) mit konkaver Kontaktfläche aufweist, welches geeignet ist mit der konkaven Kontaktfläche an der Cornea des Auges (1) anzuliegen,
wobei bei der Scanvorrichtung die optischen Elemente der Eingangs- und Fokussieroptik (6, 6", 8, 8', 8") über die spektrale Breite der von der Strahlungsquelle abgegebenen Femtosekundenpulse so chromatisch korrigiert sind, daß durch sie durchtretende fs-Pulse mit einer dispersionsbedingten zeitlichen Verbreiterung von weniger als 30 % fokussiert werden können und wobei bei der Scanvorrichtung das relativ zu der Ablenkeinrichtung bewegbare optische Element ein diffraktiv-optisches Element ist.

4. Laserchirurgisches System mit einer Scanvorrichtung nach einem der vorhergehenden Ansprüche, mit einer bevorzugt als Linearantrieb (14) ausgeführten Antriebseinrichtung zur Bewegung des relativ zu der Ablenkeinrichtung (7, 7', 7") bewegbaren ersten optischen Elements (11).

5. Laserchirurgisches System mit einer Scanvorrichtung nach einem der vorhergehenden Ansprüche, bei der die Ablenkeinrichtung (7, 7', 7") zwischen der Eingangsoptik (6, 6") und der Fokussieroptik (8, 8', 8") angeordnet ist.

6. Laserchirurgisches System mit einer Scanvorrichtung nach einem der vorhergehenden Ansprüche, bei der die Ablenkeinrichtung (7, 7', 7'') zwei voneinander beabstandete, relativ zueinander und zu der Fokussieroptik (8, 8', 8") bewegbare reflektive Elemente (9, 9') aufweist.

7. Laserchirurgisches System mit einer Scanvorrichtung nach Anspruch 6, bei der zwischen den reflektiven Elementen (9, 9') eine bevorzugt als Sammellinse (19) ausgebildete Pupillenoptik angeordnet ist, die das erste reflektive Element (9) auf das zweite reflektive Element (9') abbildet.

8. Laserchirurgisches System mit einer Scanvorrichtung nach einem der vorhergehenden Ansprüche, bei der die Fokussieroptik (8, 8', 8") eine feste Brennweite aufweist.

9. Laserchirurgisches System mit einer Scanvorrichtung nach einem der vorhergehenden Ansprüche, bei der in dem Strahlengang zwischen der Ablenkeinrichtung (7, 7') und einer Austrittslinse (15, 15') oder Austrittslinsengruppen der Fokussieroptik (8, 8') ein Strahlteiler (16, 16') angeordnet ist.

10. Laserchirurgisches System mit einer Scanvorrichtung nach Anspruch 9, bei der die Austrittslinse (15, 15') oder die Austrittslinsengruppe ein paralleles Strahlenbündel (3) in das vorgegebene Volumen fokussiert.

11. Laserchirurgisches System mit einer Scanvorrichtung nach einem der vorhergehenden Ansprüche, bei der die Fokussieroptik (8') ein Eintrittsobjektiv (20) zur Erzeugung eines reellen Zwischenbildes einer das Strahlenbündel (3) abgebenden Strahlungsquelle (2) aufweist.

12. Laserchirurgisches System mit einer Scanvorrichtung nach einem der vorhergehenden Ansprüche, bei der alle reellen Zwischenbilder einer das Strahlenbündel (3, 3") abgebenden Strahlungsquelle (2, 22) in Gas, insbesondere Luft, oder Vakuum liegen.

13. Laserchirurgisches System mit einer Scanvorrichtung nach einem der vorhergehenden Ansprüche, bei der das relativ zu der Ablenkeinrichtung (7, 7') bewegbare optische Element (11) über eine so große Strecke relativ zu der Ablenkeinrichtung (7, 7') bewegbar ist, daß der Fokus des Strahlenbündels (3) in Strahlrichtung in einen Bereich größer als 0,5 mm bewegbar ist.

14. Laserchirurgisches System mit einer Scanvorrichtung nach einem der vorhergehenden Ansprüche, bei der die Ablenkeinrichtung (7, 7') so ausgebildet ist, daß der Fokus des Strahlenbündels (3) in dem vorgegebenen Volumen in einem Bereich in einer lateralen Ebene bewegbar ist, der einen Durchmesser von 11 mm hat.

15. Laserchirurgisches System mit einer Scanvorrichtung nach einem der vorhergehenden Ansprüche mit einer als Femtosekundenlaser ausgebildeten Strahlungsquelle (2) zur Abgabe Femtosekundenlaserstrahlpulsen entlang des Strahlenbündels (3), bei der die Strahlungsquelle (2), die Eingangsoptik (6) und die Fokussieroptik (8, 8') so ausgelegt sind, daß das fokussierte Strahlenbündel (3) im Fokus einen Durchmesser kleiner als 5 Mikrometer hat.

## Claims

1. System for laser surgery with a radiation source (2) in the form of a femtosecond laser and a scanning apparatus (4) for focussing a beam (3, 3") into specified regions of the cornea of an eye (1), comprising an input optical unit (6, 6"), which is entered first by the beam (3, 3") and which has at least one first optical element (11), a focussing optical unit (8, 8', 8"), by means of which the beam (3, 3") that has emerged from the input optical unit (6, 6") is able to be focussed, and, arranged between the first optical element (11) and the focussing optical unit (8, 8', 8"), a deflection device (7, 7', 7") for deflecting the beam (3, 3") after passing the first optical element (11) in accordance with a position to be set of the focus in the lateral direction, wherein at least the one optical element (11) of the input optical unit (6, 6") is able to be moved relative to the deflection device (7, 7', 7") for the purposes of setting the position of the focus of the beam (3, 3") in the direction of the beam, wherein the system has a contact glass (5) with a concave contact surface, the contact glass being suitable for resting against the cornea of the eye (1) with the concave contact surface, wherein, in the case of the scanning apparatus, the optical elements of the input and focussing optical units (6, 6", 8, 8', 8") are chromatically corrected over the spectral width of the femtosecond pulses emitted by the radiation source such that fs pulses passing therethrough are focussed by the focussing optical unit with a dispersion-related temporal broadening of less than 30% and wherein, in the case of the scanning device, the input optical unit (6, 6') comprises a lens or lens group with negative refractive power as the first optical element (11) and the input optical unit moreover comprises a convergent lens (12) or lens group with positive refractive power downstream thereof in the direction of the beam (3, 3").

2. System for laser surgery with a scanning apparatus according to Claim 1, wherein the divergence of the beam (3, 3") emerging from the input optical unit (6, 6") is able to be altered by moving the first optical element (11), which is able to be moved relative to the deflection device (7, 7', 7"), relative to the deflection device (7, 7', 7").

3. System for laser surgery with a radiation source (2) in the form of a femtosecond laser and a scanning apparatus (4) for focussing a beam (3, 3") into specified regions of the cornea of an eye (1), comprising an input optical unit (6, 6"), which is entered first by the beam (3, 3") and which has at least one first optical element (11), a focussing optical unit (8, 8', 8"), by means of which the beam (3, 3") that has emerged from the input optical unit (6, 6") is able to be focussed, and, arranged between the first optical element (11) and the focussing optical unit (8, 8', 8"), a deflection device (7, 7', 7") for deflecting the beam (3, 3") after passing the first optical element (11) in accordance with a position to be set of the focus in the lateral direction, wherein the first optical element (11) of the input optical unit (6, 6")for setting the position of the focus of the beam (3, 3") in the direction of the beam is able to be moved relative to the deflection device (7, 7', 7"), wherein the divergence of the beam (3, 3") emerging from the input optical unit (6, 6") is able to be altered by moving the first optical element (11), which is able to be moved relative to the deflection device (7, 7', 7"), relative to the deflection device (7, 7', 7"), wherein the system has a contact glass (5) with a concave contact surface, the contact glass being suitable for resting against the cornea of the eye (1) with the concave contact surface, wherein, in the case of the scanning apparatus, the optical elements of the input and focussing optical units (6, 6", 8, 8', 8") are chromatically corrected over the spectral width of the femtosecond pulses emitted by the radiation source such that fs pulses passing therethrough can be focussed by the focussing optical unit with a dispersion-related temporal broadening of less than 30% and wherein, in the case of the scanning apparatus, the first optical element, which is able to be moved relative to the deflection device, is a diffractive-optical element.

4. System for laser surgery with a scanning apparatus according to any one of the preceding claims, comprising a drive device embodied as a linear drive (14) for moving the first optical element (11) which is able to be moved relative to the deflection device (7, 7', 7").

5. System for laser surgery with a scanning apparatus according to any one of the preceding claims, wherein the deflection device (7, 7', 7") is arranged between the input optical unit (6, 6") and the focussing optical unit (8, 8' , 8").

6. System for laser surgery with a scanning apparatus according to any one of the preceding claims, wherein the deflection device (7, 7', 7") has two spaced apart reflective elements (9, 9') that are able to be moved relative to one another and relative to the focussing optical unit (8, 8', 8").

7. System for laser surgery with a scanning apparatus according to Claim 6, wherein a pupil optical unit which images the first reflective element (9) on the second reflective element (9') and which is preferably embodied as a converging lens (19) is arranged between the reflective elements (9, 9').

8. System for laser surgery with a scanning apparatus according to any one of the preceding claims, wherein the focussing optical unit (8,', 8") has a fixed focal length.

9. System for laser surgery with a scanning apparatus according to any one of the preceding claims, wherein a beam splitter (16, 16') is arranged in the beam path between the deflection device (7, 7') and an exit lens (15, 15') or exit lens groups of the focussing optical unit (8, 8').

10. System for laser surgery with a scanning apparatus according to Claim 9, wherein the exit lens (15, 15') or the exit lens group focusses a parallel beam (3) into the specified volume.

11. System for laser surgery with a scanning apparatus according to any one of the preceding claims, wherein the focussing optical unit (8') has an entrance objective (20) for generating a real intermediate image of a radiation source (2) that emits the beam (3).

12. System for laser surgery with a scanning apparatus according to any one of the preceding claims, wherein all real intermediate images of a radiation source (2, 22) that emits the beam (3, 3") are located in a gas, in particular air, or in vacuo.

13. System for laser surgery with a scanning apparatus according to any one of the preceding claims, wherein the optical element (11) which is able to be moved relative to the deflection device (7, 7') is able to be moved over such a long path relative to the deflection device (7, 7') that the focus of the beam (3) in the beam direction is able to be moved over a range greater than 0.5 mm.

14. System for laser surgery with a scanning apparatus according to any one of the preceding claims, wherein the deflection device (7, 7') is embodied in such a way that the focus of the beam (3) is able to be moved in the specified volume in a region in a lateral plane which has a diameter of 11 mm.

15. System for laser surgery with a scanning apparatus according to any one of the preceding claims, comprising a radiation source (2) which is embodied as a femtosecond laser and which serves to output femtosecond laser beam pulses along the beam (3), wherein the radiation source (2), the input optical unit (6) and the focussing optical unit (8, 8') are designed in such a way that the focussed beam (3) has a diameter of less than 5 micrometres at the focus.

## Revendications

1. Système de chirurgie au laser doté d'une source de rayonnement (2) sous la forme d'un laser femtoseconde et d'un dispositif de balayage (4) destiné à focaliser un faisceau de rayons (3, 3") sur des zones prédéfinies de la cornée d'un œil (1), comprenant une optique d'entrée (6, 6") dans laquelle le faisceau de rayons (3, 3") pénètre en premier et qui présente au moins un premier élément optique (11), une optique de focalisation (8, 8', 8") qui permet de focaliser le faisceau de rayons (3, 3") ayant émergé de l'optique d'entrée (6, 6"), et un dispositif de déviation (7, 7', 7") disposé entre le premier élément optique (11) et l'optique de focalisation (8, 8', 8"), destiné à dévier le faisceau de rayons (3, 3") après son passage par le premier élément optique (11), selon une position à régler du foyer dans la direction latérale, dans lequel, pour le réglage de la position du foyer du faisceau de rayons (3, 3") en direction du faisceau de rayons, au moins ledit un élément optique (11) de l'optique d'entrée (6, 6") est mobile par rapport au dispositif de déviation (7, 7', 7"), le système présentant un verre de contact (5) à surface de contact concave, qui est adapté pour s'appliquer contre la cornée de l'œil (1) par la surface de contact concave, dans lequel, sur le dispositif de balayage, les éléments optiques de l'optique d'entrée et de focalisation (6, 6", 8, 8', 8") sont corrigés chromatiquement sur la largeur spectrale des impulsions femtosecondes émises par la source de rayonnement de telle sorte que des impulsions fs qui la traversent sont focalisées par l'optique de focalisation avec un élargissement temporel dû à la dispersion inférieur à 30 %, et dans lequel, sur le dispositif de balayage, l'optique d'entrée (6, 6") comprend comme premier élément optique (11) une lentille ou un groupe de lentilles d'indice de réfraction négatif, et l'optique d'entrée comprend en outre une lentille convergente (12) ou un groupe de lentilles d'indice de réfraction positif, placé(e) en aval de celle-ci/celui-ci en direction du faisceau de rayons (3, 3").

2. Système de chirurgie au laser doté d'un dispositif de balayage selon la revendication 1, dans lequel un déplacement du premier élément optique (11), mobile par rapport au dispositif de déviation (7, 7', 7"), par rapport au dispositif de déviation (7, 7', 7") permet de faire varier la divergence du faisceau de rayons (3, 3") émergeant de l'optique d'entrée (6, 6").

3. Système de chirurgie au laser doté d'une source de rayonnement (2) sous la forme d'un laser femtoseconde et d'un dispositif de balayage (4) destiné à focaliser un faisceau de rayons (3, 3") sur des zones prédéfinies de la cornée d'un œil (1), comprenant une optique d'entrée (6, 6") dans laquelle le faisceau de rayons (3, 3") pénètre en premier et qui présente au moins un premier élément optique (11), une optique de focalisation (8, 8', 8") qui permet de focaliser le faisceau de rayons (3, 3") ayant émergé de l'optique d'entrée (6, 6"), et un dispositif de déviation (7, 7', 7") disposé entre le premier élément optique (11) et l'optique de focalisation (8, 8', 8"), destiné à dévier le faisceau de rayons (3, 3") après son passage par le premier élément optique (11) selon une position à régler du foyer dans la direction latérale,
dans lequel, pour le réglage de la position du foyer du faisceau de rayons (3, 3") en direction du faisceau de rayons, le premier élément optique (11) de l'optique d'entrée (6, 6") est mobile par rapport au dispositif de déviation (7, 7', 7"), dans lequel le déplacement du premier élément optique (11), mobile par rapport au dispositif de déviation (7, 7', 7"), par rapport au dispositif de déviation (7, 7', 7") permet de faire varier la divergence du faisceau de rayons (3, 3") émergeant de l'optique d'entrée (6, 6"),
le système présentant un verre de contact (5) ayant une surface de contact concave, qui est adapté pour s'appliquer contre la cornée de l'œil (1) par la surface de contact concave, dans lequel, sur le dispositif de balayage, les éléments optiques de l'optique d'entrée et de focalisation (6, 6", 8, 8', 8") sont corrigés chromatiquement sur la largeur spectrale des impulsions femtosecondes émises par la source de rayonnement de telle sorte que des impulsions fs qui la traversent peuvent être focalisées avec un élargissement temporel dû à la dispersion inférieur à 30 %, et dans lequel, pour le dispositif de balayage, le premier élément optique, mobile par rapport au dispositif de déviation, est un élément optique de diffraction.

4. Système de chirurgie au laser doté d'un dispositif de balayage selon l'une quelconque des revendications précédentes, comprenant un dispositif d'entraînement réalisé sous la forme d'un entraînement linéaire (14), destiné à déplacer le premier élément optique (11) mobile par rapport au dispositif de déviation (7, 7', 7") .

5. Système de chirurgie au laser doté d'un dispositif de balayage selon l'une quelconque des revendications précédentes, dans lequel le dispositif de déviation (7, 7', 7") est disposé entre l'optique d'entrée (6, 6") et l'optique de focalisation (8, 8', 8").

6. Système de chirurgie au laser doté d'un dispositif de balayage selon l'une quelconque des revendications précédentes, dans lequel le dispositif de déviation (7, 7', 7") présente deux éléments réfléchissants (9, 9') espacés l'un de l'autre, mobiles l'un par rapport à l'autre et par rapport à l'optique de focalisation (8, 8', 8").

7. Système de chirurgie au laser doté d'un dispositif de balayage selon la revendication 6, dans lequel l'optique pupillaire, réalisée de préférence sous la forme d'une lentille convergente (19), est disposée entre les éléments réfléchissants (9, 9') et reproduit le premier élément réfléchissant (9) sur le deuxième élément réfléchissant (9').

8. Système de chirurgie au laser doté d'un dispositif de balayage selon l'une quelconque des revendications précédentes, dans lequel l'optique de focalisation (8, 8', 8") présente une focale fixe.

9. Système de chirurgie au laser doté d'un dispositif de balayage selon l'une quelconque des revendications précédentes, dans lequel un séparateur de faisceau (16, 16') est disposé sur la trajectoire des rayons entre le dispositif de déviation (7, 7') et une lentille de sortie (15, 15') ou des groupes de lentilles de sortie de l'optique de focalisation (8, 8').

10. Système de chirurgie au laser doté d'un dispositif de balayage selon la revendication 9, dans lequel la lentille de sortie (15, 15') ou le groupe de lentilles de sortie focalise un faisceau de rayons parallèle (3) sur le volume prédéfini.

11. Système de chirurgie au laser doté d'un dispositif de balayage selon l'une quelconque des revendications précédentes, dans lequel l'optique de focalisation (8') présente un objectif d'entrée (20) destiné à produire une image intermédiaire réelle d'une source de rayonnement (2) émettant le faisceau de rayons (3).

12. Système de chirurgie au laser doté d'un dispositif de balayage selon l'une quelconque des revendications précédentes, dans lequel toutes les images intermédiaires réelles d'une source de rayonnement (2, 22) émettant le faisceau de rayons (3, 3") se trouvent dans un gaz, en particulier dans l'air, ou sous vide.

13. Système de chirurgie au laser doté d'un dispositif de balayage selon l'une quelconque des revendications précédentes, dans lequel l'élément optique (11), mobile par rapport au dispositif de déviation (7, 7'), est mobile par rapport au dispositif de déviation (7, 7') sur une distance telle que le foyer du faisceau de rayons (3) est mobile dans la direction des rayons dans une plage supérieure à 0,5 mm.

14. Système de chirurgie au laser doté d'un dispositif de balayage selon l'une quelconque des revendications précédentes, dans lequel le dispositif de déviation (7, 7') est réalisé de telle sorte que le foyer du faisceau de rayons (3) est mobile dans le volume prédéfini dans une zone dans un plan latéral qui présente un diamètre de 11 mm.

15. Système de chirurgie au laser doté d'un dispositif de balayage selon l'une quelconque des revendications précédentes, comprenant une source de rayonnement (2) réalisée sous la forme d'un laser femtoseconde, destinée à émettre des impulsions de rayonnement laser femtoseconde le long du faisceau de rayons (3), dans lequel la source de rayonnement (2), l'optique d'entrée (6) et l'optique de focalisation (8, 8') sont conçues de telle sorte que le faisceau de rayons focalisé (3) au foyer présente un diamètre inférieur à 5 micromètres.
